# EUROPEAN PATENT APPLICATION

(11) **EP 2 774 979 A1**
(43) Date of publication of application: **10.09.2014**
(21) Application number: 13157703.3
(22) Date of filing: 04.03.2013
(51) Int. Cl.: C12M 1/00, C12M 1/34, C12M 1/36, G01N 27/26, G01N 27/28, G01N 33/00

(54) **Bioreactor and sensing system therefor**

(71) Applicant: Gymetrics SA, 1024 Ecublens (CH)
(72) Inventor: Ferguson, Michael, 1275 Cheserex (CH); Jaber, Karim, 1209 Geneve (CH)
(74) Representative: reuteler & cie SA

(57) **Abstract**

The present invention relates to a measurement probe system (6) comprising a reusable unit, a pluggable electrical connector (12) and a disposable, single-use sensing unit (14), the reusable unit comprising a connector support (27), the disposable, single-use sensing unit (14) comprising a sealing sleeve (32) and a sensing device (36). The sensing device (36) is configured to be fixedly connected to the sealing sleeve (32). The pluggable electrical connector (12) comprises a connecting portion (28) configured to be connected to the connector support (27) and a complementary connecting portion (30), which is connected to the sensing device (36) and to the sealing sleeve (32). The complementary connecting portion (30) is configured to interact with the connecting portion (28) to provide electronic communication between the sensing device (36) and the reusable unit, whereby the sealing sleeve (32) of the single-use sensing unit (14) is configured to removably receive the connector support (27) with the connecting portion (28).

## Description

The present invention relates to a bioreactor and to a system for measuring parameters in a bioreactor.

Bioreactors used in the biotechnology or pharmaceutical technology field, which may typically contain 3 to 5 liters or more of culture medium, are usually equipped with one or more probes that each measure a certain parameter relevant to growth of a culture in the bioreactor. The measured parameters typically include temperature and pH of the culture medium and carbon dioxide content in the bioreactor. Probes measuring parameters in the culture medium have a sensor arranged at or close to the bottom of the bioreactor. The probes are mounted to a top enclosing wall of the bioreactor and comprise pluggable electrical connectors accessible from the outer side of the top wall for connection to complementary connectors at ends of cables connected to a computer work station for recording and monitoring the measured parameters.

Reusable bioreactors have a containing wall that is usually made of glass or stainless steel that is sterilizable by autoclave. It is known to provide certain disposable single-use container parts for bioreactors, such container parts made of a heat sterilizable plastic material. Nevertheless, the sensor probes and other components in contact with the culture medium are reusable and need to be cleaned and sterilized before each use such that the benefits of being able to dispose of certain container parts is limited.

Conventional sensor probes typically comprise a stainless steel tube housing the parameter measurement sensor, connecting wires and electronics, the tube extending from the top wall of the bioreactor towards the bottom end of the bioreactor whereby the sensing element is arranged at the bottom end of the probe. The steel tube protects the electronic parts and sensor from the cleaning and sterilizing process for re-use. The cleaning and sterilizing steps are however time consuming and thus costly.

An object of the present invention is to provide a measurement probe for a bioreactor that is sterile and reliable, yet is economical to use.

Another object of the present invention is to provide a bioreactor with a system for the measurement of parameters in a culture medium that is versatile and effective, yet economical to use.

It is advantageous to provide a measurement probe that is compact and economical to manufacture.

It is advantageous to provide a bioreactor system and components thereof that are easy and economical to sterilize.

Objects of the invention have been achieved by providing a measurement probe system according to claim 1 for a bioreactor.

Objects of the invention have been achieved by providing a bioreactor according to claim 14.

Disclosed herein is a measurement probe system for a bioreactor including a reusable unit and a disposable single-use sensing unit. The reusable unit comprises a rigid connector support and a pluggable electrical connector mounted on the connector support for connection to a measurement signal processing unit. The single-use sensing unit comprises a sealing sleeve hermetically sealing an inside of the sealing sleeve from an outside of the sealing sleeve configured to be in contact with a culture medium in a container portion of said bioreactor. A complementary electrical connector is positioned in said inside of said sealing sleeve configured for releasable connection to the pluggable electrical connector, and a sensing device fixedly mounted to an outside of the sealing sleeve configured for contact with said culture medium, the sealing sleeve being configured to removably receive the pluggable electrical connector therein.

The sealing sleeve may advantageously be made of one or more polymers selected from the group consisting of Polycarbonate (PC), Polyvinylchloride (PVC), Polyethersulfone (PES), Polyetherimide (PEI), Polyetheretherketone (PEEK) and Polysulfone (PS) that are resistant to one or more of the common biological sterilization processes.

In an embodiment, the sensing device comprises a circuit board with a bio-reaction parameter sensor mounted thereon. The sensor circuit board may comprise material selected from liquid crystal polymers (LCP), LCP hybrids, ceramic, hybrid ceramics and Polyimide laminates that are resistant to one or more of the common biological sterilization processes.

The sensing device may advantageously be anchored to a free end of the sealing sleeve with a sealing potting material.

The pluggable electrical connector may, in an embodiment, advantageously comprise a plurality of spring biased contact pins and the complementary electrical connector may comprise electrical contact pads mounted on a face of an insulating support.

The insulating support may, in an embodiment, be in the form of a substrate of a printed circuit board (PCB) and the contact pads in the form of metal deposited surfaces formed on the PCB.

The reusable unit may advantageously integrate said measurement signal processing unit, the measurement signal processing unit being mounted on an end of the connector support distal from said pluggable electrical connector. The measurement signal processing unit may advantageously comprise an electronic processing circuit and a graphical user interface that provides data on a measured bioreactor parameter. The measurement signal processing unit may advantageously comprise an autonomous power source and a wireless communications system.

The connector support may comprise a rigid hollow tube within which a cable or other conductors extend to connect the sensing device to the measurement probe system.

The single-use sensing unit may comprise a sensor housing portion extending from an end of the sealing sleeve and configured to protect the sensing device.

The insulating support of the complementary electrical connector hermetically closes the end of the sealing sleeve.

Also disclosed herein is a bioreactor including a disposable single-use bioreactor housing comprising a coupling interface and container portion for receiving a culture medium therein, and at least one sealing sleeve of a measurement probe system assembled to the coupling interface, the bioreactor housing and sealing sleeve forming disposable single use components.

The coupling interface may be configured to receive and fixedly hold a plurality of said sealing sleeves for measuring multiple parameters relevant to the bioreaction process.

The bioreactor housing may be made of one or more polymers that are biologically sterilizable by known methods such as autoclave, gamma radiation, or electron beam, such polymers including Polycarbonate (PC), Polyvinylchloride (PVC), Polyethersulfone (PES), Polyetherimide (PEI), Polyetheretherketone (PEEK) and Polysulfone (PS).

Further objects and advantageous features of the invention will be apparent from the claims, from the detailed description, and annexed drawings, in which:
Figure 1 shows an illustrative view of a bioreactor system according to an embodiment of the invention;
Figure 2 shows a perspective partially exploded view of a measurement probe system according to an embodiment of the invention;
Figure 3 shows another perspective partially exploded view of the measurement probe system of figure 2;
Figure 4 shows another perspective view of the measurement probe system of figure 2;
Figure 5 shows a perspective view of a bioreactor system according to an embodiment of the invention with a portion of the container of the bioreactor cut away to better view the interior;
Figure 6 shows a perspective schematic view of a reusable electrical connector portion in contact with a complementary disposable single-use connector portion of a sensing portion of the measurement probe system according to an embodiment of the invention;
Figure 7 shows a similar perspective view as figure 6 but with the disposable complementary connector portion disconnected from the reusable connector portion; and
Figure 8 shows a view of a cross section of an embodiment of a sensing portion of a the measurement probe system according to an embodiment of the invention.

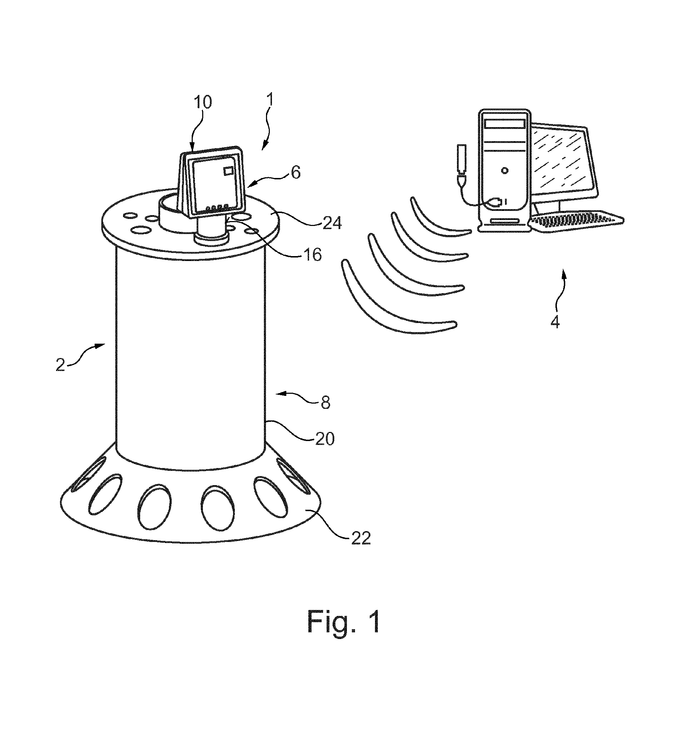

Referring to figure 1, an exemplary embodiment of a bioreactor system 1 comprises a bioreactor 2 and a computer work station 4.

The bioreactor 2 comprises a housing 8 and a measurement probe system 6.

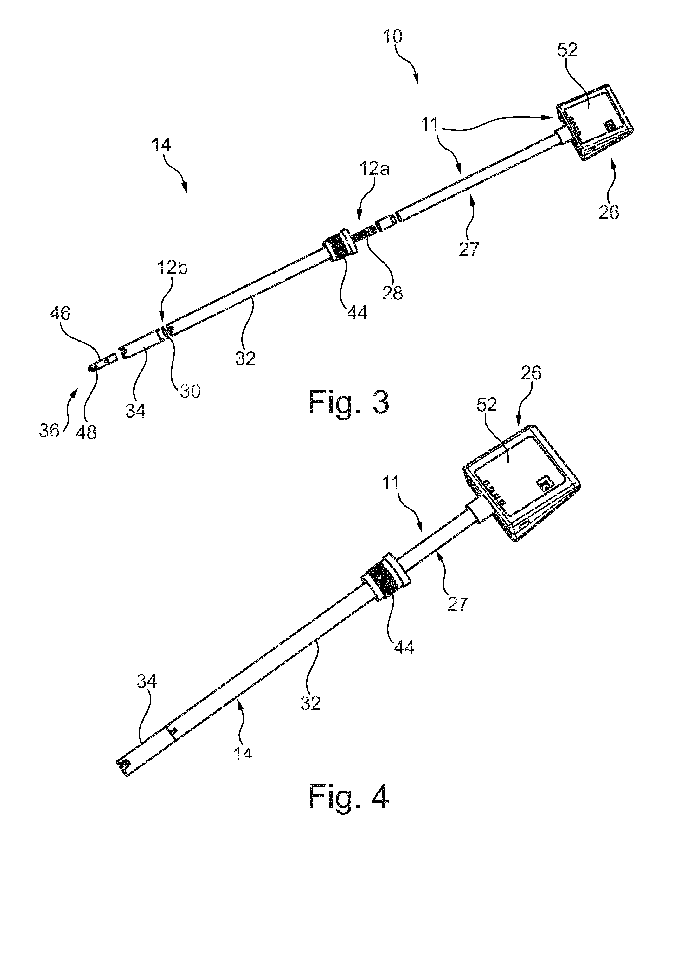

The measurement probe system 6 comprises a reusable unit 10 and a disposable single-use sensing unit 14, as best shown in figures 3 to 5.

The bioreactor housing 8 may be in the form of a conventional reusable bioreactor housing, or according to an embodiment of the invention, in the form of a disposable single-use bioreactor housing 8. In an embodiment, the disposable single-use sensing unit 14 and the disposable single-use bioreactor housing 8 form together a disposable, single unit to which one or more reusable units 10 of one or more measurement probe systems 6 may be removably assembled.

The bioreactor 2 may comprise a support base 22, which is configured to embed and support a container portion 20 of the bioreactor housing 8. The container portion 20 is configured to receive a cell culture medium therein.

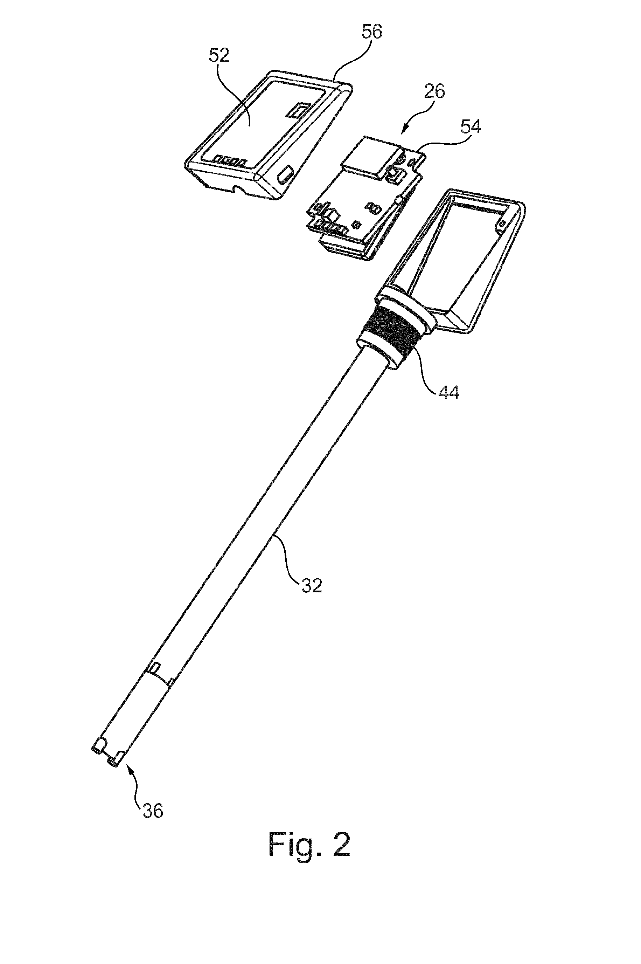

The reusable unit 10 of the measurement probe system is configured to be removably assembled to the single-use sensing unit 14 as best illustrated in figures 2 to 5.

The reusable unit 10 comprises a measurement signal processing unit 26 and a sensor connection portion 11. The measurement signal processing unit may comprise a housing 56, a user interface 52, for example in the form of a screen and/or control buttons, an autonomous power source, preferably a rechargeable battery, and an electronic processing circuit 54 configured to capture and process the measurement signals received from the sensor and further including a communications circuit for communicating data to an external device, in particular a computer work station 4. The autonomous power source may also include means to scavenge power from the environment, for instance light and/or RF (radio frequency) or other electromagnetic signals.

The communications circuit is preferably configured to communicate data wirelessly to the work station. The data communication may be one-way only, from the measurement probe to the work station, or in a variant, two-way between the probe and the work station. In a variant, the wireless data communication may be effected by RFID (Radio Frequency Identification) communication technology whereby the measurement signal processing unit 26 is provided with an RFID chip that responds to RF signals emitted by the workstation or other external device. The latter solution advantageously lowers the power consumption and power requirements of the measurement signal processing unit 26. In variants, other known wireless communications systems may be used, in particular near-field communications systems including Bluetooth and Zigbee or midfield such as Wi-LAN. Within the scope of the invention it is also possible to incorporate far field telecommunications systems in the communications circuit, such as GSM or other mobile telecommunications standards.

The measurement signal processing unit 26 may be fixedly connected to the sensor connection portion 11. In an advantageous variant the measurement signal processing unit 26 is removably connected to the sensor connection portion 11 by means of a pluggable or releasable connector mechanism (not shown). In the latter variant, connector supports of different configurations, for instance different lengths, diameters or shapes, or even sensor devices of other configurations, may be connected to measurement signal processing units 26 having a common configuration.

The sensor connection portion 11 comprises a pluggable or otherwise releasable electrical connector 12 and a support 27 that may comprise a rigid tube, for instance made of a as metal or a rigid polymer or a composite material, configured to receive therein electrical conductors (not shown), for example in the form of wires or cables, interconnecting the measurement signal processing unit 26 to the connector 12.

The disposable, single-use unit 14 comprises a sealing sleeve 32 configured to receive the sensor connection portion 11 of the reusable unit 10 therein, as best illustrated in figures 2 to 5. The sealing sleeve 32 is configured to be mounted and fixed to a coupling interface 24 of the bioreactor housing 8.

The coupling interface of a conventional bioreactor housing forms a removable reusable lid for the container portion 20 and has a plurality of ports 74, 74' allowing screw mounting of measurement probes to the lid, access for a mixer and for a valve to regulate pressure in the bioreactor. According to an embodiment of the invention, the measurement probe system 6 comprises a conventional coupling portion 44 allowing mounting of the system to a conventional bioreactor lid.

The sealing sleeve 32 comprises a coupling portion 44 configured to couple the sealing sleeve to a complementary port 74 in the coupling interface 24 of the bioreactor housing 8. The coupling portion 44 may be a thread or it may comprise a latching mechanism, which allows clicking the sealing sleeve 32 into the coupling interface 24. An O-ring seal or other sealing means may be provided between the port 74 and coupling portion 44 to ensure hermetic sealing therebetween The coupling portion 44 is arranged at a top end region of the sealing sleeve 32.

According to an advantageous aspect of the invention, the sealing sleeve 32 may be pre-assembled to a disposable single use coupling interface 24, configured to be assembled to or supplied as a kit with a disposable single use container portion 20. In the latter embodiment all the bioreactor components entering into contact with the culture medium are thus disposable single use articles thus avoiding operations of cleaning and sterilizing the bioreactor after use. The sealing sleeve, container portion and coupling interface may be made of moulded plastic parts in a polymer material resistant to the sterilization process during manufacturing or prior to use. The sealing sleeve or plurality of sealing sleeves, depending on the variant, may be integrally formed with the coupling interface, or unremovably fixed to the coupling interface, for instance by welding or bonding, to form a single component, thus reducing assembly operations in situ before the bioreaction process.

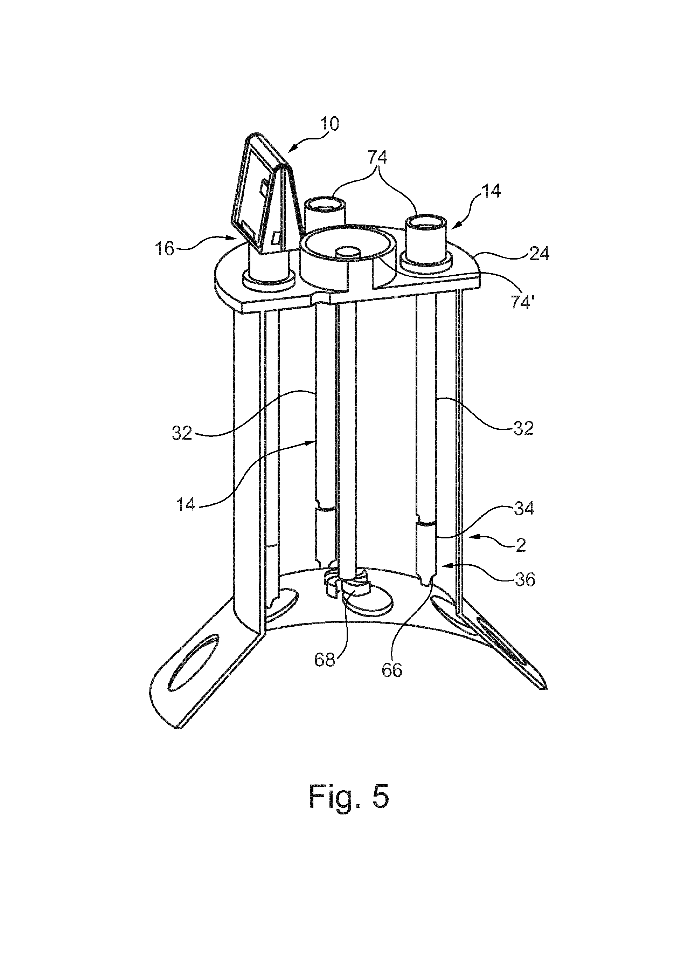

As illustrated in figure 5, the coupling interface 24 of the bioreactor housing 8 comprises various ports 74, to receive single-use sensing units 14. A port 74' may be configured to hold a mixing device 68 to mix the cell culture medium.

The sealing sleeve 32 is configured to extend into the container portion 20 of the bioreactor housing 8.

One of the commonly found bioreactor sizes found in industry receives 3 to 5 liters of culture medium. Bioreactors of various sizes may however be provided, for instance by varying the height of the container portion 20 while maintaining the same diameter in order to mount common coupling interfaces 24, and/or by varying the diameter of the container portion.

Between the sealing sleeve 32 and the coupling interface 24 a sealing such as an O-ring for example made of rubber or resin sealing may be applied to avoid that the inside of the bioreactor housing 8 can establish physical contact with the outside of the bioreactor housing 8.

At the end region arranged opposite the coupling portion 44, a sensing device 36 is arranged. The sensing device 36 comprises a bio-reaction parameter sensor 48 configured to measure a parameter relevant to the bio-reaction process, which may for instance be a cell culture process. Parameters relevant to the bio-reaction process may include for example:
- Temperature
- pH
- (dissolved) O₂ content
- (dissolved) CO₂ content

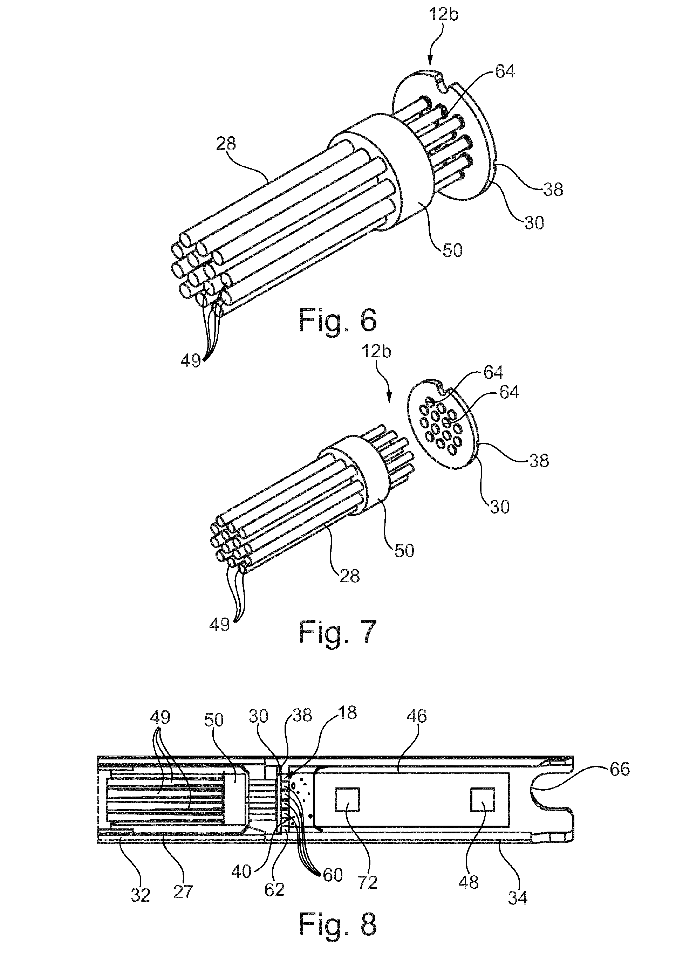

Various sensors based on optical or electrical detection means for measuring the above parameters are known *per* se and may be employed in the present invention. As best illustrated in figures 3 and 8, the sensing device 36 may comprise a circuit board 46, for instance a printed circuit board (PCB), and a micro controller unit 72 for processing measurement signals from the sensor 48 prior to transmission to the measurement signal processing unit 26.

The sensing device 36 is electrically connected via an unpluggable or otherwise releasable or disconnectable electrical connector system 12a, 12b to the reusable unit 10, as best illustrated in figures 3, 6, 7 and 8. The releasable electrical connector system comprises a pluggable electrical connector 12a mounted to the connector support 27 of the reusable unit 10, and a complementary connector portion 12b mounted to disposable single use sensing unit 14.

The complementary connector portion 12b is, in an advantageous embodiment, arranged close to an end of the sealing sleeve 32 distal from the measurement signal processing unit 26.

In the embodiment illustrated, the complementary connector portion 12b advantageously comprises electrical terminals in the form of contact pads 64 mounted on a face of an insulating support 30. The insulating support may advantageously be in the form of a printed circuit board with the electrical contact pads 64 formed thereon and interconnected by conductive circuit traces or plated through holes to an opposite side of the insulating support for connection to the sensor 48. The sensing device 36 may be connected to the electrical terminals 64 of the complementary electrical connector 12b by various means, including a pluggable connection or a solder or welded connection. In the embodiment shown, the sensing device comprises a circuit board 46 comprising electrical terminals 60 in the form of contact pads arranged along an edge of the circuit board 46, said edge mounted against the insulating support 30 such that the contact pads 60 are in electrical contact with complementary circuit pads of the complementary connector portion 12b. In the embodiment shown in figures 6 to 8, the insulating support 30 comprises a slot 38 for lodging the edge of the sensor circuit board 46. The electrical connection between contact terminals on the insulating support and the sensor circuit board may be effected by a clamping contact or by a permanent connection such as soldering or welding. In the embodiment illustrated, the sensor circuit board 46 and the circuit board forming the insulating support 30 are mounted orthogonally with respect to each other.

In the embodiment illustrated in figure 8, the insulating support 30 of the complementary electrical connector advantageously closes the end of the sealing sleeve 32, such that the interior of the sealing sleeve 32 receiving the connector of the reusable unit 10 is hermetically sealed from the interior of the bioreactor receiving the culture medium. The hermetic sealing may be ensured or reinforced by a layer of insulating sealing or potting material 40 received in the sensor housing portion 34 and covering a side of the insulating support 30 connected to the sensing device 36. The potting material 40, which for instance may be an epoxy resin, may also serve to reinforce the anchoring of the sensing device 36 to the end of the sealing sleeve 32.

In a variant as illustrated in the figures, the disposable, single-use sensing unit 14 may comprise a sensor housing portion 34 configured to protect the sensing device 36. The sensor housing portion may have various shapes and sizes depending on the nature and geometry of the sensing device received therein. The sensor housing portion 34 may be integrally formed with an end of the sealing sleeve 32 as a single part, for instance a single molded or extruded plastic part, or as in the variant illustrated, as a separately manufactured part that is permanently assembled to the sealing sleeve, for instance by bonding or welding the sensor housing portion to the end of the sealing sleeve. The latter embodiment advantageously allows the sensing device to be preassembled more easily and have different shapes and sizes for mounting to common interface of a sealing sleeve.

The sensor housing portion 34 may comprise an inner shoulder element 62 configured to abut the insulating support 30 and thereby position the complementary electrical connector 12b with respect to the electrical terminals 28 of the pluggable connector 12a. The insulating support 30 may be fixed to the inner shoulder element 62 via a bonding material, or by welding, or by a latching protuberance (not shown).

The sensor housing portion 34 may be configured to be fixedly connected to the sealing sleeve 32 via epoxy resin or any other type of sealed connection. Alternatively the sensor housing portion 34 may be connected to the sealing sleeve 32 by welding, such as for example ultrasonic welding.

In a variant, the inner shoulder element 62 may be formed in the sealing sleeve 32. In the latter variant the separate sensor housing portion 34 may be omitted and the sealing sleeve 32 may provide an overlapping protection (not shown) to the sensing device 36.

In an advantageous embodiment, the sensor housing portion 34 comprises one or a plurality of cut outs 66 at its free end as best illustrated in figure 8. These cut outs 66 enhance the mixing and flow of the culture medium within the sensor housing portion 34 around the sensor 48, thus improving contact between the sensor 48 and a homogenized culture medium.

The sensor housing portion 34 is configured to protect the sensing device 36 from damage and external influences, however within the scope of the invention it is also envisageable to have a sensing device that is not surrounded by a housing portion and thus projects partially or totally from the end of the sealing sleeve 32 without protection.

The sealing sleeve 32, the sensing device 36, the sensor housing portion 34 (if present) and the complementary electrical connector 12b advantageously form a disposable, single-use sensing unit 14.

The single-use sensing unit 14 is configured to receive the sensing portion of the reusable unit 10, comprising the connector support 27 and the releasable electrical connector 12a and to form a hermetic separation between the reusable components and the culture medium contained in the bioreactor container portion 20.

The pluggable connector 12a of the reusable unit comprises electrical terminals 28 as shown schematically in figures 6 to 8, the terminals connected to the electrical conductors (not shown) extending through the connector support 27 for interconnection to the measurement signal processing unit 26.

The electrical terminals 28 may comprise a plurality of pins contact 49, which are configured to establish an electronic connection with the complementary electrical terminals 64 of the complementary electrical connector 12b. The connecting pins 49 may advantageously be biased by springs (not shown) mounted in the connector housing 50 to ensure a good electrical pressing contact against the pad shaped terminal 64. On the other end the contact pins 49 are fixedly connected to conductors of a cable that provides electrical communication with the measurement signal processing unit 26.

The connector 12a is mounted at an end of the connector support 27 that, for instance, may comprise a hollow tube. The housing 50 of the connector 12a is fixed to the connector support be permanent means such as crimping, welding or bonding, or alternatively by releasable means such as a screw thread coupling, bayonet coupling or latching coupling to allow repair, replacement or maintenance of the electrical connector 12a.

Since the reusable unit 10 is reusable it is possible to provide multiple single-use sensing units 14 extending into the bioreactor container portion 20, each with different sensing devices for measuring different parameters, and a single reusable unit 10 or a plurality of reusable units less than the number of single use sensing units. Also, it is possible to have a plurality of bioreactors in use and to share reusable sensing units between the plurality of bioreactors.

The reusable unit 10 may be configured to be connected to single-use sensing units 14 comprising different parameter sensing portions 36, such as illustrated in figure 5. The reusable unit 10 may be used for various single-use bioreactors 8 equipped with single-use sensing units 14. The use of a comparable small amount of reusable units 10 with a high amount of single-use sensing units 14 saves costs and improves efficiency.

The reusable unit 10 may be plugged into the disposable single-use sensing unit 14 by inserting the sensor connection portion 11 into the sealing sleeve 32, whereby the sealing sleeve and connector 12a and/or connector support 27 are provided with interengaging polarizing means (not shown), for instance a protuberance on one part and a complementary groove on the other part, that are configured to ensure correct orientation of the connector 12a with respect to the complementary connector 12b when the reusable unit is fully plugged into the single-use sensing unit. The reusable unit 10 and single-use sensing unit 14 may further comprise interengaging releasable latching mechanism (not shown) or similar means to lock the two units together during use.

The outer diameter of the connector support 27 is only slightly smaller than the inner diameter of the sealing sleeve 32 to ensure accurate and stable mounting of the reusable unit 10 to the single-use sensing unit 14. The connector support and the sealing sleeve need not necessarily be cylindrical in shape and may have various other cross-sectional profiles shapes including elliptical, rectangular, square, polygonal or irregular forms. The term "diameter" used herein is intended to indicate generally the cross-sectional size and does not automatically imply a circular cross-section.

The disposable, single-use sensing unit 14 and, in certain embodiments also the single-use bioreactor housing 8 are configured to be used once and then thrown away. The single-use sensing unit 14 and the single-use bioreactor housing 8 are preferably made of polymer materials, except for the electronic, electrical and active parts of the sensing device and connector, configured to withstand the most common sterilizing processes such as gamma radiation, autoclave and electron beam (E-beam) processing yet allow low cost manufacturing and assembly as well as low negative environmental impact after use.

All the expensive parts of the measurement probe system 6 may be embedded in the reusable unit 10. Since there is no need to sterilize the reusable unit 10, the operation of a bioreaction process with the devices of the invention is very economical and safe.

As illustrated in figure 5, the bioreactor housing 8 may be provided as a single-use unit, preferably made of a molded polymer material, assembled to one or more single-use sensing units 14 to form together a disposable single-use bioreactor container and sensing unit, which is configured to be sterilized during the manufacturing process, or alternatively *in situ* before use, by one of the above mentioned common sterilizing processes. The single-use bioreactor sensing unit is configured to be used as a bioreactor sensing kit together with one more reusable units 10.

The measurement probe system 6 is preferably configured to communicate wirelessly with a work station 4 that gathers and processes measurement data on the biological processes in the bioreactor to monitor and control the processes. It is of course also possible to connect the measurement probe system 6 to external computing equipment via cables. The measurement probe system 6 however advantageously may be provided with an electronic processing circuit 54 and a graphical user interface (GUI) 52 that provides data on the measured parameter to the operator without the operator having to consult the work station. Further information such as the state of charge of the autonomous power source in the measurement processing unit 26, and alarms signaling the deviation of a parameter beyond a specified threshold, may also be provided via the GUI.

According to a variant, the bioreactor system may be provided with a docking station (not shown) with a connector system configured to connect to the connector 12a, the docking station configured for one or more functions selected from recharging the autonomous power source in the measurement probe system 6, downloading of measurement data stored in a memory of the measurement probe system 6, resetting or calibration of the measurement probe system 6, and updating or reprogramming of the electronic processing circuit of the measurement probe system 6.

The disposable components of embodiments of a bioreactor system according to this invention are sterilizable by one or more of biological sterilization methods including autoclave, gamma radiation and electron beam. The materials from which the disposable components are made and that support at least one of these sterilization processes include those listed hereafter.

The sealing sleeve 32 and the sensor housing portion 34 may be made of or comprise one or more of polycarbonate (PC), polyvinylchloride (PVC), polyethersulfone (PES), polyetherimide (PEI), polyetheretherketone (PEEK), and polysulfone (PS).

The sealing sleeve 32 and the sensor housing portion 34 may be made of the same material, or of different materials.

The sensor circuit board 46 and the insulating support 30 may be made of or comprise one or more of liquid crystal polymers (LCP), LCP hybrids, ceramics, hybrid ceramics and polyimide laminates.

## Claims

1. Measurement probe system (6) for a bioreactor including a reusable unit (10) and a disposable single-use sensing unit (14), the reusable unit comprising a rigid connector support (27) and a pluggable electrical connector (12a) mounted on the connector support for connection to a measurement signal processing unit (26), the single-use sensing unit comprising a sealing sleeve (32) hermetically sealing an inside of the sealing sleeve from an outside of the sealing sleeve configured to be in contact with a culture medium in a container portion (20) of said bioreactor, a complementary electrical connector (12b) positioned in said inside of said sealing sleeve configured for releasable connection to the pluggable electrical connector, and a sensing device (36) fixedly mounted to an outside of the sealing sleeve configured for contact with said culture medium, the sealing sleeve being configured to removably receive the pluggable electrical connector therein.

2. Measurement probe system according to claim 1, wherein the sealing sleeve (32) is made of one or more polymers selected from the group consisting of Polycarbonate (PC), Polyvinylchloride (PVC), Polyethersulfone (PES), Polyetherimide (PEI), Polyetheretherketone (PEEK) and Polysulfone (PS).

3. Measurement probe system according to claim 1 or 2, wherein the sensing device comprises a circuit board (46) with a bio-reaction parameter sensor (48) mounted thereon.

4. Measurement probe system according to the preceding claim, wherein the sensor circuit board comprises material selected from liquid crystal polymers (LCP), LCP hybrids, ceramic, hybrid ceramics and Polyimide laminates.

5. Measurement probe system according to any of the preceding claims, wherein the sensing device is sealingly anchored to a free end of the sealing sleeve with a potting material (40).

6. Measurement probe system according to any of the preceding claims, wherein the pluggable electrical connector comprises a plurality of spring biased contact pins (49).

7. Measurement probe system according to the preceding claim, wherein the complementary electrical connector (12b) comprises electrical contact pads (64) mounted on a face of an insulating support (30).

8. Measurement probe system according to the preceding claim wherein the insulating support is in the form of a substrate of a printed circuit board (PCB) and the contact pads are in the form of metal deposited surfaces formed on the PCB.

9. Measurement probe system according to any of the preceding claims, wherein the reusable unit comprises said measurement signal processing unit, the measurement signal processing unit being mounted on an end of the connector support distal from said pluggable electrical connector.

10. Measurement probe system according to the preceding claim wherein the measurement probe system comprises an electronic processing circuit (54), an autonomous power source, a wireless communications system, and a graphical user interface (52) that provides data on a measured bioreaction parameter.

11. Measurement probe system according to any of the preceding claims, wherein the connector support comprises a rigid hollow tube.

12. Measurement probe system according to any of the preceding claims, wherein the single-use sensing unit comprises a sensor housing portion (34) extending from an end of the sealing sleeve and configured to protect the sensing device.

13. Measurement probe system according to any of the preceding claims, wherein an insulating support (30) of the complementary electrical connector hermetically closes the end of the sealing sleeve.

14. Bioreactor (2) comprising a disposable single-use bioreactor housing (8) comprising a coupling interface (24) and container portion (20) for receiving a culture medium therein, and at least one sealing sleeve of a measurement probe system according to any one of the preceding claims assembled to the coupling interface, the bioreactor housing and sealing sleeve forming a disposable single use entity.

15. Bioreactor according to the preceding claim wherein the coupling interface is configured to receive and fixedly hold a plurality of said sealing sleeves.

16. Bioreactor according to claim 14 or 15, wherein the bioreactor housing is made of one or more polymers selected from the group consisting of Polycarbonate (PC), Polyvinylchloride (PVC), Polyethersulfone (PES), Polyetherimide (PEI), Polyetheretherketone (PEEK) and Polysulfone (PS).

17. Bioreactor according to claim 14, 15, or 16 further comprising a reusable unit of a measurement probe system according to any one of claims 1 to 13.
